(19) Europäisches Patentamt
European Patent Office
Office européen des brevets

(11) **EP 2 127 677 A1**

(12) **EUROPEAN PATENT APPLICATION**
published in accordance with Art. 153(4) EPC

(43) Date of publication:
**02.12.2009 Bulletin 2009/49**

(51) Int Cl.:
***A61K 47/32*** (2006.01)

(21) Application number: **07860227.3**

(22) Date of filing: **26.12.2007**

(86) International application number:
**PCT/JP2007/074998**

(87) International publication number:
**WO 2008/081829 (10.07.2008 Gazette 2008/28)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **27.12.2006 US 877165 P**

(71) Applicant: **Astellas Pharma Inc.**
**Tokyo 103-8411 (JP)**

(72) Inventors:
• **YOSHIDA, Takatsune**
**Tokyo 103-8411 (JP)**
• **YOSHIHARA, Keiichi**
**Tokyo 103-8411 (JP)**
• **UMEJIMA, Hiroyuki**
**Tokyo 103-8411 (JP)**
• **KURIMOTO, Ippei**
**Tokyo 103-8411 (JP)**

(74) Representative: **Gille Hrabal Struck Neidlein Prop Roos**
**Patentanwälte**
**Brucknerstrasse 20**
**40593 Düsseldorf (DE)**

(54) **AMINOALKYLMETHACRYLATE COPOLYMER E FOR MAINTAINING SOLUBILITY OF POORLY WATER-SOLUBLE DRUG**

(57) A pharmaceutical composition comprising aminoalkyl methacrylate copolymer E, an acidic substance, and a poorly-soluble drug, wherein the aminoalkyl methacrylate copolymer E and the acidic substance are uniformly mixed, and solubility of the poorly-soluble drug is maintained for at least 30 minutes is disclosed.

EP 2 127 677 A1

**Description**

TECHNICAL FIELD

The present invention relates to a pharmaceutical composition characterized by comprising

(1) a poorly-soluble drug and
(2) aminoalkyl methacrylate copolymer E which is uniformly mixed with an acidic substance,

wherein solubility of the poorly-soluble drug is maintained for at least 30 minutes; and
a use of aminoalkyl methacrylate copolymer E which is uniformly mixed with an acidic substance, in the manufacture of a pharmaceutical composition for inhibiting reprecipitation of a solubilized poorly-soluble drug.

BACKGROUND ART

Poorly-soluble drugs generally exhibit a low absorption in a human, due to a low solubility in water. Solubilization of poorly-soluble drugs has been attempted by various approaches, such as solubilization using organic solvents, surfactants, oils, or the like, amorphization (solid dispersion), nanoparticulation, or the like.
Solubilization using organic solvents is mainly applied to injections, and ethanol, propylene glycol, and the like are typical solvents. As the solubilization using surfactants, oils, or the like, for example, oily preparations, liquid preparations, soft capsules, and the like are typically exemplified. In the nanoparticulation technique, a poorly-soluble drug is dry- or wet-pulverized in the presence of a polymer or a surfactant so as to reduce the particle size of poorly-soluble drug to submicron. In this technique, an increased surface area of the poorly-soluble drug enhances the dissolution rate of the poorly-soluble drug.
In the solid dispersion technique, a typical technique of amorphization, a solid dispersion is prepared by a method in which a poorly-soluble drug and a polymer substances are dissolved in a solvent and spray-dried, a method in which a poorly-soluble drug is dissolved in a polymer substance by heating (melting, a melt extrusion), or the like. An amorphized poorly-soluble drug can be dissolved more rapidly in comparison with a crystalline form, and temporarily accomplishes a highly solubilized state (supersaturated state).
For example, patent reference 1 discloses a solid dispersion in which a water-insoluble ionic polymer, such as methacrylate copolymer S, methacrylate copolymer L, aminoalkyl methacrylate copolymer E, or the like, is used as a carrier to prevent the crystallization of a drug in a powder state.
However, in all of the above-mentioned methods, the supersaturated state of a drug is temporarily achieved in an aqueous solution, but the drug is gradually reprecipitated and the solubility is lowered. Further, a poorly-soluble drug which tends to be crystallized is reprecipitated immediately after being solubilized, and thus, there is still room for improvement from the viewpoints of a low supersaturation and a low degree of effectiveness in enhancing solubility.
Patent reference 2 discloses a technique for improving solubility using a concentration-enhancing polymer, as a technique which improves the solubility of a drug by a solid dispersion and enhances the concentration of the drug after dissolution. In this technique, a polymer used as the concentration-enhancing polymer is hydroxypropylmethylcellulose, but a further improvement is desired, because of a low degree of effectiveness in inhibiting reprecipitation.
Nonpatent reference 1 discloses a melting technique in which aminoalkyl methacrylate copolymer E and ethyl acrylate/methyl methacrylate copolymer are combined. This invention can inhibit the reprecipitation of a poorly-soluble drug by a combination of aminoalkyl methacrylate copolymer E and ethyl acrylate/methyl methacrylate copolymer. However, the enhanced solubility of the drug is caused by the ethyl acrylate/methyl methacrylate copolymer, and nonpatent reference 1 does not disclose that aminoalkyl methacrylate copolymer E having an enhanced solubility can inhibit the reprecipitation of a poorly-soluble drug.
Patent reference 3 discloses a technique for enhancing absorption of a drug by aminoalkyl methacrylate copolymer E and an acid substance. This invention can enhance the absorption of a drug by inhibiting an interaction between the drug and substances located on the gastrointestinal mucosa and/or the mucous layer. Patent reference 3 does not disclose nor suggest that aminoalkyl methacrylate copolymer E having a solubility enhanced by uniformly mixing it with an acidic substance can inhibit the reprecipitation of a poorly-soluble drug.
As described above, instead of such techniques for temporarily enhancing the solubility of a poorly-soluble drug, a convenient technique for maintaining the enhanced solubility for a long time and accomplishing an efficient absorption from the gastrointestinal tract, by inhibiting the reprecipitation of a poorly-soluble drug dissolved, is desired.
[patent reference 1] Japanese Unexamined Patent Publication (Kokai) No. 2000-95708
[patent reference 2] Japanese Translation Publication (Kohyo) No. 2005-500313

[patent reference 3] WO 02/05786

[non-patent reference 1] Kathrin Nollenberger (Degussa, Pharma Polymers, Darmstadt, Germany) et al., "Increasing the solubility of Felodipine by preparing solid dispersions with melt extrusion", 2006, No.1240, Controlled release society

DISCLOSURE OF INVENTION

PROBLEMS TO BE SOLVED BY THE INVENTION

An object of the present invention is to inhibit reprecipitation of a poorly-soluble drug which becomes supersaturated in an aqueous solution by various solubilizing treatments, and to maintain an improved solubility. Another object is to reduce variability of absorption when a poorly-soluble drug is administered, and to maintain sufficient absorption.

To enhance the absorption of a poorly-soluble drug, the present inventors examined various solubilizing treatments, and examined whether reprecipitation of a solubilized poorly-soluble drug was inhibited, and whether the supersaturated state thereof in an aqueous solution was maintained. When hydroxypropylmethylcellulose, polyvinylpyrrolidone, aminoalkyl methacrylate copolymer E, and the like, which are known as conventional substances for preparing a solid dispersion, were used to prepare various solid dispersions, every substance improved the solubility of a poorly-soluble drug, but the supersaturated state was not maintained for a long time.

Aminoalkyl methacrylate copolymer E, which is not usually used together with acid because it is a gastro-soluble substance, was dissolved by adding an acid thereto, and the resulting solution was added to a dissolution fluid around neutrality which was the pH condition at the upper gastrointestinal tract from which drugs were absorbed. As a result, it was found that the solubilized state of a poorly-soluble drug was maintained, and that its high solubility was constantly maintained for a long time. Next, aminoalkyl methacrylate copolymer E having an enhanced solubility, which had been prepared by spray-drying or lyophilizing aminoalkyl methacrylate copolymer E solubilized with acid, was used to prepare a solid dispersion of a poorly-soluble drug, and its effect in maintaining the solubilized state thereof was further examined. As a result, it was found that solubility approximately 60 times higher than that of a solid dispersion using hydroxypropylmethylcellulose could be maintained for 24 hours, and the present invention was completed.

MEANS FOR SOLVING THE PROBLEMS

The present invention relates to

1. a pharmaceutical composition comprising aminoalkyl methacrylate copolymer E, an acidic substance, and a poorly-soluble drug, wherein the aminoalkyl methacrylate copolymer E and the acidic substance are uniformly mixed, and solubility of the poorly-soluble drug is maintained for at least 30 minutes;

2. the pharmaceutical composition of 1, wherein the poorly-soluble drug is an amorphous state;

3. the pharmaceutical composition of 1, wherein the poorly-soluble drug is a solid dispersion;

4. the pharmaceutical composition of 1, wherein the poorly-soluble drug is dissolved in a solvent selected from an organic solvent, a surfactant, or an oily substance, or a mixture of one, or two or more of the solvents;

5. the pharmaceutical composition of 1, wherein the poorly-soluble drug is tacrolimus;

6. use of aminoalkyl methacrylate copolymer E which is uniformly mixed with an acidic substance, in the manufacture of a pharmaceutical composition for inhibiting reprecipitation of a poorly-soluble drug;

7. use of aminoalkyl methacrylate copolymer E which is uniformly mixed with an acidic substance of 6, wherein the poorly-soluble drug is solubilized;

8. a method of solubilizing a poorly-soluble drug, by using aminoalkyl methacrylate copolymer E which is uniformly mixed with an acidic substance;

9. a method of inhibiting reprecipitation of a poorly-soluble drug, by using aminoalkyl methacrylate copolymer E which is uniformly mixed with an acidic substance;

10. a method of maintaining a maximum percentage dissolved of a poorly-soluble drug within 90% to 100% for at least 30 minutes, when (1) a dissolution test is carried out by adding, to a dissolution test fluid, aminoalkyl methacrylate copolymer E which is uniformly mixed with an acidic substance, followed by a solubilized poorly-soluble drug or a dissolved poorly-soluble drug, or (2) a dissolution test is carried out by adding aminoalkyl methacrylate copolymer E which is uniformly mixed with an acidic substance, and a solid dispersion containing a poorly-soluble drug, to a dissolution test fluid; and

11. a process of manufacturing a pharmaceutical composition for maintaining solubility and/or a solubilized state of a poorly-soluble drug for at least 30 minutes, comprising the steps of:

preparing aminoalkyl methacrylate copolymer E which is uniformly mixed with an acidic substance by a method selected from:

> (1) a method in which the acidic substance and the aminoalkyl methacrylate copolymer E are dissolved in water or a solvent, and the obtained liquid is spray-dried,
> (2) a method in which the acidic substance and the aminoalkyl methacrylate copolymer E are dissolved in water or a solvent, and the obtained liquid is lyophilized, or
> (3) a method in which the acidic substance and the aminoalkyl methacrylate copolymer E are dissolved in water or a solvent, and the obtained liquid is dried by evaporating the water or solvent therefrom, and mixing the aminoalkyl methacrylate copolymer E with a solubilized poorly-soluble drug or a dissolved poorly-soluble drug.

EFFECTS OF THE INVENTION

According to the present invention, a composition containing a poorly-soluble drug having an enhanced solubility by various solubilizing techniques and aminoalkyl methacrylate copolymer E uniformly mixed with an acidic substance is used to inhibit reprecipitation of the solubilized poorly-soluble drug in a solution, and thereby the solubility of the poorly-soluble drug and the absorption thereof from the gastrointestinal tract can be enhanced.

BRIEF DESCRIPTION OF THE DRAWINGS

[Figure 1] Figure 1 is a graph showing the result of a reprecipitation test for compound A in Experimental Example 1.
[Figure 2] Figure 2 is a graph showing the result of a reprecipitation test for compound A in Experimental Example 2.
[Figure 3] Figure 3 is a graph showing the result of a reprecipitation test for compound A in Experimental Example 3.
[Figure 4] Figure 4 is a graph showing the result of a reprecipitation test for compound A in Experimental Example 4.
[Figure 5] Figure 5 is a graph showing the result of a reprecipitation test for compound B in Experimental Example 5.
[Figure 6] Figure 6 is a graph showing the result of a reprecipitation test for tacrolimus in Experimental Example 6.
[Figure 7] Figure 7 is a graph showing the result of a reprecipitation test for compound A in Experimental Example 7.
[Figure 8] Figure 8 is a graph showing absorption of tacrolimus in rats in Experimental Example 10.
[Figure 9] Figure 9 is a graph showing absorption of tacrolimus in dogs in Experimental Example 11.

BEST MODE FOR CARRYING OUT THE INVENTION

The solubility of a poorly-soluble drug in the present invention may be determined by, for example, the following methods.
As a conventional method for determining the solubility of a drug, a method based on a UV absorption spectrum of a drug may be used. Solubility can be calculated from absorbance, because solubility of a drug is proportional to an absorbance at an absorption wavelength inherent to the drug. A method of measuring an absorbance by adding a solution to a cell, a method of measuring an absorbance by using a high performance liquid chromatography, or the like may be exemplified.
The maintenance of solubility of a drug may be determined by sequentially measuring the solubility of the drug with a dissolution tester. For example, solubility can be measured in accordance with a dissolution test, method 2 (paddle method), described in the Japanese Pharmacopoeia, at a paddle rotation speed of 50 rpm, at a constant temperature of 37°C. More particularly, a solution of a drug dissolved with an organic solvent is added to 500 mL of a second fluid for disintegrating test used in a disintegrating test described in the Japanese Pharmacopoeia fifteenth edition containing 50 mg of additives. Samples are taken at regular intervals, and a drug absorption wavelength of each sample is measured to determine the maintenance of solubility of a drug.
Instead of the Japanese Pharmacopoeia, similar test methods described in various Pharmacopoeias of other countries, including the European Pharmacopoeia, the United States Pharmacopoeia, and the like may be used.
The improvement of solubility or the inhibition of reprecipitation as used herein means that the improvement of solubility of a poorly-soluble drug and the maintenance of the solubilized state for a long time. The term "a long time" as used herein means a period of time for which the solubility of a poorly-soluble drug is maintained, as described below.
The term "to maintain the solubility of a poorly-soluble drug for at least 30 minutes" as used herein means that the solubility of a poorly-soluble drug is maintained for 30 minutes or more, on the basis of a time showing a maximum percentage dissolved, when the solubility is measured in accordance with one of the test methods as exemplified above. The duration of time that the solubility is maintained is not particularly limited, so long as it is for 30 minutes or more, but, for example, the time lapsed until the reprecipitation of a poorly-soluble drug begins is preferably 1 hour or more, more preferably 2 hours or more, most preferably 12 hours or more. The term "maintain (the solubility)" generally means not to lower the solubility. However, since the degree depends on solubility or a concentration of a poorly-soluble drug, it should be appropriately determined according to a poorly-soluble drug, and is not particularly limited. For example, the term "maintain" as used herein means not to lower the solubility to less than 50% of a maximum percentage dissolved of a poorly-soluble drug, when the solubility is measured by a dissolution test. This lower limit is preferably 80%, more preferably 90%.

In the case that aminoalkyl methacrylate copolymer E which is uniformly mixed with an acidic substance is not contained, or such a composition is used, a decreased percentage dissolved is observed when the solubility of a poorly-soluble drug is measured. The term "to maintain the solubility of a poorly-soluble drug for at least 30 minutes" as used herein includes to maintain the solubility for 30 minutes or more, with respect to the beginning time of the reduction.

The term "to inhibit reprecipitation" as used herein means to maintain the solubility of a poorly-soluble drug.

The term "poorly-soluble drug" as used herein means a drug having a low solubility in water. The poorly-soluble drug is not particularly limited, so long as it is an active ingredient effective in treatment or prevention. For example, a drug having a water solubility of 1,000 $\mu$g/mL or less, preferably 100 $\mu$g/mL or less, more preferably 10 $\mu$g/mL or less, most preferably 1 $\mu$g/mL or less, may be exemplified.

As the drugs, there may be mentioned, for example, drugs for the circulatory system, such as isosorbid nitrate, nifedipine, felodipine, barnidipine hydrochloride, nicardipine hydrochloride, indenolol hydrochloride, furosemide, spironolactone, guanetidine nitrate, reserpine, amosulalol hydrochloride, lisinopril, methoprolol, pilocarbpine or the like, anti-inflammatory agents, such as indomethacin, ketoprofen, or the like, steroids, such as hydrocortisone, danazol, prednisolone, or the like, analgesics, such as naproxen or the like, anticonvulsant agents, such as carbamazepine or the like, antiinfectants, such as ketoconazole, immunosuppressive agent, such as tacrolimus, cyclosporine, or the like, BPH (Benign Prostatic Hyperplasia) therapeutic agents, such as tamsulosin hydrochloride or the like, antiasthmatics, such as theophylline or the like, agents for improving peripheral circulation, such as prostaglandin I derivatives such as beraprost sodium or the like, antithrombotics, hypotensives, agents for treatment of heart failure, agents for treatment of various complications of diabetes, agents for treatment of peptic ulcer, agents for treatment of skin ulcers, agents for treatment of hyperlipidemia, or the like.

Among these drugs, tacrolimus, N-(2,6-Dimethylphenyl)-N-(2-{[4-(1,2,4-oxadiazol-3-yl)phenyl]amino}-2-oxoethyl)tetrahydro-2H-thiopyran-4-carboxamide 1,1-dioxide (hereinafter referred to as compound A), and a 1,4,5,6-tetrahydroimidazo[4,5-d]benzoazepine derivative or a salt thereof (hereinafter referred to as compound B) are preferred. Tacrolimus is a macrolide immunosuppressant which was discovered at a Lead Discovery Laboratories of Astellas Pharma Inc. (former name: Fujisawa Pharmaceutical. Co., Ltd.) in 1984. Tacrolimus is a poorly-soluble drug having a water solubility of 1 $\mu$g/mL or less, and the present invention enables it to have an enhanced absorption.

These drugs may be used in a free form, or as a pharmaceutically acceptable salt. These drugs may be used alone, or in a combination of two or more drugs. The content of aminoalkyl methacrylate copolymer E having an enhanced solubility by using acid, with respect to the drug, is not particularly limited, so long as the solubility and/or the solubilized state of a poorly-soluble drug can be maintained for a long time. For example, when the poorly-soluble drug is tacrolimus, the content of aminoalkyl methacrylate copolymer E having an enhanced solubility is 1 or more parts, preferably 3 or more parts, more preferably 5 or more parts, with respect to 1 part of tacrolimus.

Poorly-soluble drugs as used in the present invention include a poorly-soluble drug dissolved in a solvent, a poorly-soluble drug solubilized by various treatments, and the like. As such treatments for solubilization, a method of forming crystalline nanoparticles, a method of forming a solid dispersion, and the like may be exemplified.

The crystalline nanoparticles as used herein means crystalline microparticles having a particle size of 1 $\mu$m or less, preferably 300 nm or less, prepared by pulverization or the like to enhance a rate of dissolution. In general, crystalline nanoparticles may be prepared, for example, by dispersing a poorly-soluble drug in a solution in which a polymer and/or a surfactant is dissolved, and pulverizing the resulting dispersion with a wet pulverizer to obtain the poorly-soluble drug as particles having a particle size of approximately 100 to 300 nm. As the pulverizer, a bead mill using zirconia beads or polystyrene beads, a high-pressure homogenizer not using a pulverizing medium, or the like may be used. Alternatively, nanoparticles may be prepared by dry pulverizing a poorly-soluble drug with a polymer and/or a surfactant.

The solid dispersion as used herein means a state in which an amorphous poorly-soluble drug is contained in an appropriate carrier. In general, a poorly-soluble drug is dissolved in an organic solvent, a carrier is dissolved or dispersed, and then, the solvent is removed to obtain a solid dispersion. As the carrier, polymers such as polyvinylpyrrolidone, copolyvidone, polyvinyl alcohol, hydroxypropylmethylcellulose, or the like, may be generally used. Further, aminoalkyl methacrylate copolymer E having an enhanced solubility with acid may be used.

As a method of removing a solvent in the present invention, for example, heating, spray-drying, melting, lyophilization, kneading, and the like may be exemplified. Further, a dry pulverizing method may be used to form an amorphous poorly-soluble drug, without an organic solvent, and prepare a solid dispersion.

The spray-drying as used in the present invention means a method in which a solution of a poorly-soluble drug dissolved with a carrier such as a polymer or the like is spray-dried with an apparatus such as a spray-dryer, a fluidized bed granulator, or the like. A powder in which an amorphous poorly-soluble drug is dispersed in a carrier such as a polymer or the like may be obtained by instantaneously evaporating an organic solvent.

The melting as used in the present invention means a method in which a substance such as a polymer, wax, or the like is heated, a poorly-soluble drug is added to the melted substance and dissolved therein, and the mixture is solidified by cooling. A powder in which an amorphous poorly-soluble drug is dispersed in a substance may be obtained. This method can be carried out by using a twinscrew extruder or the like.

The lyophilization as used in the present invention means a method in which a solution prepared by dissolving a poorly-soluble drug and various additives is frozen, and a solution is evaporated and removed by, for example, drying under reduced pressure or the like. A powder in which an amorphous poorly-soluble drug is dispersed in a carrier such as a polymer or the like may be obtained by evaporating a solution.

The kneading as used in the present invention is a method in which a solution prepared by dissolving a poorly-soluble drug in an organic solvent or the like is added to a carrier such as a polymer or the like, the whole is well kneaded, and the solvent is removed from the mixture to prepare a solid dispersion. In this method, an insoluble carrier is generally used. A solvent may be removed by, for example, drying by ventilation, drying under reduced pressure, or the like.

The dry pulverizing method as used in the present invention is a method in which a poorly-soluble drug is mixed and pulverized with mixed powder of a polymer and/or various additives for pulverization (such as sugars, inorganic substances, or the like) using a dry pulverizer, to obtain a powder in which an amorphous poorly-soluble drug is dispersed in a polymer substance. As the dry pulverizer, a ball mill, a pin mill, a jet mill, or the like may be used.

The aminoalkyl methacrylate copolymer E in the present invention is a copolymer of methyl methacrylate, butyl methacrylate, and dimethylaminoethyl methacrylate (chemical name). A commercially available product, Eudragit E (Degussa), may be used.

The aminoalkyl methacrylate copolymer E having an enhanced solubility used in the present invention is a compound in which a dissolution rate thereof in an aqueous solution under acidic or neutral conditions is improved by protonating the tertiary amines in dimethylaminoethyl moieties of the aminoalkyl methacrylate copolymer E with acid. The term "aminoalkyl methacrylate copolymer E which is uniformly mixed with an acidic substance" as used herein means aminoalkyl methacrylate copolymer E having an enhanced solubility.

The acidic substance which may be used in the present invention is not particularly limited, so long as it is pharmaceutically acceptable and capable of dissolving the aminoalkyl methacrylate copolymer E by neutralizing some or all of the basic groups of the copolymer in the presence of water. As the acidic substance, an inorganic acid and/or an organic acid and/or a polymer in which the pH of a solution prepared by dissolving or suspending 1 g of the substance in 50 mL of water is 6 or lower are preferred. As the acidic substance, there may be mentioned, for example, inorganic acids (such as hydrochloric acid, sulfuric acid, phosphoric acid, potassium dihydrogen phosphate, sodium dihydrogen phosphate, or the like), organic acids (such as citric acid, lactic acid, tartaric acid, fumaric acid, phthalic acid, propyl gallate, acetic acid, oxalic acid, malonic acid, adipic acid, phytic acid, succinic acid, glutaric acid, maleic acid, malic acid, mandelic acid, ascorbic acid, benzoic acid, methanesulfonic acid, capric acid, capronic acid, caprylic acid, lauric acid, arachidonic acid, erucic acid, linoleic acid, linolenic acid, oleic acid, palmitic acid, myristic acid, stearic acid, or the like), polymers (such as hydroxypropylmethylcellulose, povidone, copolyvidone, or the like), aspartic acid, L-glutamic acid, L-cystein, arginine hydrochloride, alginic acid, erythorbic acid, lysine hydrochloride, L-glutamic acid hydrochloride, gelatin, or the like. These acidic substances may be used alone or as a combination thereof.

In the present invention, the aminoalkyl methacrylate copolymer E which is uniformly mixed with an acidic substance may be used in the following methods, for example, a method in which the aminoalkyl methacrylate copolymer E is previously dissolved in a solution, in an in vitro test, a method in which the aminoalkyl methacrylate copolymer E is used as a substance for preparing a solid dispersion, a method in which the aminoalkyl methacrylate copolymer E is mixed with a poorly-soluble drug solubilized by various treatments, or the like.

The aminoalkyl methacrylate copolymer E which is uniformly mixed with an acidic substance may be prepared, for example, by dissolving aminoalkyl methacrylate copolymer E in dilute hydrochloric acid, and removing water from the resulting solution by spray-drying using an apparatus (such as a spray-dryer, a fluidized bed granulator, or the like), lyophilization, or the like.

The aminoalkyl methacrylate copolymer E may contain a surfactant. The surfactant to be added is not particularly limited, so long as it is pharmaceutically acceptable and is capable of reducing the water repellency of the copolymer. As the surfactant, there may be mentioned, for example, nonionic surfactants [for example, polyoxyethylene surfactants (such as polysorbate 80, polyoxyl stearate 40, lauromacrogol, polyoxyethylene-hydrogenated castor oil (HCO-60), sucrose fatty acid ester, Pluronic, and the like)], ionic surfactants [anionic surfactants (for example, sodium lauryl sulfate and the like), cationic surfactants (for example, benzalkonium chloride and the like), or amphoteric surfactants (for example, lecithin, casein, and the like)], polymer surfactants [anionic surfactants (for example, gum arabic, sodium alginate, sodium pectinate, xanthan gum, carboxymethylcellulose, carboxystarch, methacrylate copolymer, maleate copolymer, polystyrenesulfonate, and the like), cationic surfactants (for example, chitosan, cationized cellulose, alkylated cationized cellulose, cationized starch, cationized guar gum, alkylated chitosan, povidone, vinylpyridine copolymer, polyethyleneimine, and the like), nonionic surfactants (for example, corn starch, dextrin, starches, methylcellulose, ethylcellulose, hydroxyethylcellulose, hydroxypropylmethylcellulose, alkylated hydroxypropylmethylcellulose, alkylated inulin, polyvinyl alcohol, polyoxyethylene polyoxypropylene copolymer, polyoxyethylene polyisopropylene copolymer, polyvinyl ether, polyacrylamide, and the like), or amphoteric surfactants (for example, casein, methacryloyloxyethylphosphorylcholine polymer, and the like)], and the like. These surfactants may be used alone, or as a combination of two or more surfactants.

The content of the acidic substance used in the present invention is not particularly limited, so long as it is an amount capable of dissolving the aminoalkyl methacrylate copolymer E by neutralizing some or all of the basic groups of the copolymer in the presence of water. The amount of the acidic substance to be added is an amount which neutralizes approximately 1% or more, preferably approximately 5% or more, more preferably approximately 10% or more, still more preferably approximately 30% or more, most preferably 50% or more, of the basic groups of the copolymer. It is preferable that this value is 50% or more, because a spray-dried product can be easily handled during production without aggregation, even when stored for a long period of time. Further, as shown in Experimental Example 3 described below, a sufficient effect is expected even if the amount is more than 100%. The amount of the acidic substance may be appropriately adjusted in accordance with the solubility and/or the acidity of the acidic substance, and it is generally 0.005 to 50 parts by weight, preferably 0.01 to 30 parts by weight, more preferably 0.03 to 10 parts by weight, with respect to 1 part by weight of the aminoalkyl methacrylate copolymer E.

When 312.5 g of 1 mol/L hydrochloric acid is added, as the acidic substance used in the present invention, to 500 g of Eudragit E, and the mixture is spray dried, the calculation may be carried out by the following equation (I):

$$(1 \times 312.5)/1000 \ [a] = X/[KOH(56)] \ [b] \qquad (I)$$

[a: Number of moles of HCl, b: Number of moles of KOH, 56: Molecular weight of KOH]
X = 17.49 g, but it is the amount in 500 g, and thus, this value is divided by 500.
X/1 g Eudragit E = 35 mg KOH

Actually, the alkali value in 1 g of Eudragit E is 163 to 198 mg KOH, and thus, the amount of the acid added is 17.7 to 21.5% of the amount that neutralizes all of the alkali.

The pharmaceutical composition of the present invention may be formulated using one or more pharmaceutical excipients, which are not particularly limited, so long as they are pharmaceutically acceptable. Such excipients include, for example, binders, disintegrating agents, viscous agents, fillers, lubricants, stabilizers, gelatinizing agents, corrigents, flavors, acidulants, foaming agents, artificial sweeteners, coloring agents, and the like.

As the binders, there may be mentioned, for example, polyvinylpyrrolidone, hydroxypropylmethylcellulose, hydroxypropylcellulose, gum arabic, agar, guar gum, polyethylene glycol, gelatin, pullulan, polyvinyl alcohol, gelatinized starch, and the like.

As the disintegrating agents, there may be mentioned, for example, starches (such as corn starch, partially-gelatinized starch, and the like), carmellose calcium, crospovidone, low-substituted hydroxypropyl cellulose, crystalline cellulose, croscarmellose sodium, and the like

As the viscous agents, there may be mentioned, for example, sodium polyacrylate, polyethylene oxide, polycarbophil, hydroxypropylmethylcellulose, hydroxypropylcellulose, sodium alginate, propylene glycol alginate, carageenan, and the like.

As the lubricants, there may be mentioned, for example, magnesium stearate, calcium stearate, sucrose fatty acid ester, polyethylene glycol, talc, stearic acid, stearyl alcohol, and the like.

As the stabilizing agents, there may be mentioned, for example, ascorbic acid, magnesium L- aspartate, acetyltryptophan sodium, aminoethylsulfonic acid, DL-alanine, L-alanine, sodium hydrogensulfite, sodium alginate, benzoic acid, inositol, edetate sodium, erythorbic acid, sodium erythorbate, calcium chloride, sodium chloride, benzethonium chloride, magnesia chloride, cysteine hydrochloride, lysine hydrochloride, cacao butter, casein, fructose, sodium caprylate, carmellose calcium, carmellose sodium, dried sodium sulfite, dried sodium carbonate, xanthan gum, xylitol, triethyl citrate, glycine, tartaric acid, cyclodextrin, sodium acetate, magnesium oxide, potassium bicarbonate, sodium thioglycolate, potassium thiocyanate, dextran, heparin sodium, methylhesperidin, malonic acid, and the like.

As the gelatinizing agents, there may be mentioned, for example, sodium polyacrylate, polyethylene oxide, polycarbophil, hydroxypropylmethylcellulose, hydroxypropylcellulose, sodium alginate, mannan, pectin, agar, carageenan, and the like.

As the corrigents, there may be mentioned, for example, aspartame, sucrose, saccharin sodium, dipotassium glycyrrhizinate, stevia, thaumatin, citric acid, and the like.

As the flavors, there may be mentioned, for example, menthol, peppermint, lemon, lemon lime, orange, peppermint oil, and the like

As the acidulants, there may be mentioned, for example, citric acid, tartaric acid, malic acid, and the like.

As the foaming agents, there may be mentioned, for example, sodium bicarbonate and the like.

As the artificial sweeteners, there may be mentioned, for example, saccharin sodium, dipotassium glycyrrhizinate, aspartame, stevia, somatin, and the like.

As the coloring agents, there may be mentioned, for example, red ferric oxide, yellow ferric oxide, food yellow No. 4, food yellow No. 5, food blue No. 3, and the like. Any substance for coloring may be used.

These pharmaceutical excipients may be used alone or as a combination of two or more excipients in appropriate amounts. The pharmaceutical excipients as used in the present invention are not limited to the above exemplified excipients.

A solvent which may be used for dissolving a poorly-soluble drug in the present invention is not particularly limited, but there may be mentioned, for example, organic solvents (such as concentrated glycerin, propylene glycol, methanol, ethanol, 1,2,6-hexanetriol, and the like), polyethylene glycol surfactants (such as macrogol, polyoxyethylene-hydrogenated castor oil, polyoxyethylene, polyoxypropylene, lauromacrogol, polyoxyl 35 castor oil, polysorbate, polypropylene glycol, polyoxyethylene alkyl ether-stearyl alcohol mixture, polyoxyethylene alkyl ether, polyoxyethylene oleyl amine, polyoxyethylene oleyl ether, polyoxyethylene oleyl amine, polyoxyethylene oleyl ether, diethanolamine polyoxyethylene oleyl ether phosphate, sodium polyoxyethylene oleyl ether phosphate, polyoxyethylene distyrylphenyl ether, polyoxyethylene stearyl ether, polyoxyethylene stearyl ether phosphate, polyoxyethylene cetyl ether, sodium polyoxyethylene cetyl ether phosphate, polyoxyethylene sorbitan monolaurate, polyoxyethylene sorbitol beeswax, polyoxyethylene nonyl phenyl ether, polyoxyethylene castor oil, polyoxyethylene polyoxypropylene glycol, polyoxyethylene polyoxypropylene cetyl ether, polyoxyethylene coconut oil fatty acid glyceryl, polyoxyethylene lanolin, polyoxyethylene lanolin alcohol ether, and the like), ionic surfactants [anionic surfactants (for example, sodium lauryl sulfate and the like), cationic surfactants (for example, benzalkonium chloride and the like), or amphoteric surfactants (for example, lecithin, casein, and the like)], and the like. These surfactants may be used alone or as an appropriate combination of two or more surfactants.

An oily substance which may be used for dissolving a poorly-soluble drug in the present invention is not particularly limited, but there may be mentioned, for example, olive oil, soybean oil, castor oil, sunflower oil, coconut oil, sesame oil, safflower oil, corn oil, rapeseed oil, cottonseed oil-soybean oil mixture, process oil, almond oil, fatty acids (such as oleic acid, safflower oil fatty acids, isostearic acid, and the like), phospholipids (typically soybean lecithin), wax (such as cacao butter, castor wax, carnauba wax, white beeswax, beeswax, montanic acid ester wax, liquid paraffin, hard fat, and the like), higher alcohols (such as stearyl alcohol, cetanol, myristyl alcohol, cetostearyl alcohol, behenyl alcohol, lauryl alcohol, lanolin alcohol, isostearyl alcohol, and the like), fatty acid esters (such as glycerin fatty acid ester, acetylglycerin fatty acid ester, propylene glycol alginate ester, polyoxyl stearate 40, sorbitan fatty acid ester, sorbitan trioleate, tricaprylin, propylene glycol fatty acid ester, octyldodecyl myristate, sorbitan monooleate, glyceryl monostearate, sorbitan monopalmitate, sorbitan monolaurate, lipophilic glyceryl monooleate, lipophilic glyceryl monostearate, diisopropyl adipate, diisobutyl adipate, polyoxyl 45 stearate, polyoxyl 55 stearate, diethyl sebacate, glyceryl triisooctanoate, polyoxyethylene glyceryl triisostearate, triethylene glycol, sorbitan tristearate, isopropyl palmitate, cetyl palmitate, isopropyl myristate, cetyl myristate, myristyl myristate, $\alpha$-monoisostearyl glyceryl ether, glyceryl monooleate, polyethylene glycol monooleate, polyethylene sorbitan monooleate, ethylene glycol monostearate, sorbitan monostearate, butyl monostearate, propylene glycol monostearate, polyethylene glycol monostearate, polyoxyethylene glyceryl monostearate, polyoxyethylene sorbitan monostearate, glyceryl monomyristate, polyethylene glycol monolaurate, polyoxyethylene sorbitan monolaurate, decaglyceryl laurate, hexyl laurate, isopropyl laurate, ethyl linoleate, isostearyl palmitate, isostearyl hexadecyl, methyl myristate, and the like), and the like.

A preparation to which the present invention can be applied is not particularly limited, but there may be mentioned, for example, preparations for oral administration (such as tablets, capsules, powder, granules, a liquid, or the like), injections (such as ampules, vials, infusion bags, or the like), suppositories, transnasal agents, inhalation agents, transdermal administration (such as patches, ointment, or the like), or the like. Further, the present invention can be used as a combination with various formulation techniques, such as controlled release preparations as described in WO 94-006414, timed-release preparations as described in WO 01-078686, intrabuccally fast disintegrating tablets as described in WO 95-20380, or the like.

EXAMPLES

The present invention will now be further illustrated by, but is by no means limited to, the following Examples.

Example 1: Aminoalkyl methacrylate copolymer E having enhanced solubility prepared by spray-drying

Aminoalkyl methacrylate copolymer E (product name: Eudragit E, Degussa)(500 g), 10 w/v% dilute hydrochloric acid (367.5 g), and purified water (2632.5 g) were mixed, and stirred until the aminoalkyl methacrylate copolymer E was dissolved. The resulting solution was spray-dried (spraying volume: 30 g/min, inlet temperature: 145°C, rotation rate of atomizer: 20000±500 rpm) using a spray-dryer (manufactured by Ohkawara Kakohki Co., Ltd., L-8) to prepare aminoalkyl methacrylate copolymer E having a solubility enhanced with acid.

Example 2: Aminoalkyl methacrylate copolymer E having enhanced solubility prepared by spray-drying

Aminoalkyl methacrylate copolymer E (product name: Eudragit E, Degussa)(500 g), 10 w/v% dilute hydrochloric acid (367.5 g), Tween 80(Ionet T-80PA, derived from a plant, Sanyo Chemical Industries, LTD.)(50 g), and purified water (2632.5 g) were mixed, and stirred until the aminoalkyl methacrylate copolymer E was dissolved. The resulting solution was spray-dried (spraying volume: 30 g/min, inlet temperature: 145°C, rotation rate of atomizer: 20000±500 rpm) using a spray-dryer (Manufactured by Oh kawara Kakohki Co., Ltd., L-8) to prepare aminoalkyl methacrylate copolymer E having a solubility enhanced with acid.

Example 3: Aminoalkyl methacrylate copolymer E having enhanced solubility prepared by lyophilization

Aminoalkyl methacrylate copolymer E (product name: Eudragit E, Degussa)(2 g), 1N hydrochloric acid (4.1 mL), and purified water (100 mL) were mixed, and stirred until the aminoalkyl methacrylate copolymer E was dissolved. The resulting solution was lyophilized using a freeze dryer FD-81 (Tokyo Rikakikai Co,. Ltd.) to prepare aminoalkyl methacrylate copolymer E having a solubility enhanced with acid.

Example 4: Aminoalkyl methacrylate copolymer E having enhanced solubility prepared by lyophilization

Aminoalkyl methacrylate copolymer E (product name: Eudragit E, Degussa)(2 g), 1N hydrochloric acid (4.1 mL), Tween 80(Ionet T-80PA, derived from a plant, Sanyo Chemical Industries, LTD.)(0.2 g), and purified water (100 mL) were mixed, and stirred until the aminoalkyl methacrylate copolymer E was dissolved. The resulting solution was lyophilized using a freeze dryer FD-81 (Tokyo Rikakikai Co,. Ltd.) to prepare aminoalkyl methacrylate copolymer E having a solubility enhanced with acid.

Example 5: Aminoalkyl methacrylate copolymer E having enhanced solubility prepared by lyophilization

The procedure as described in Example 3 was repeated, except that the amount of hydrochloric acid was 4.9 mL, to prepare aminoalkyl methacrylate copolymer E having a solubility enhanced with acid.

Example 6: Aminoalkyl methacrylate copolymer E having enhanced solubility prepared by lyophilization

The procedure as described in Example 3 was repeated, except that the amount of hydrochloric acid was 6.0 mL, to prepare aminoalkyl methacrylate copolymer E having a solubility enhanced with acid.

Examples 7: Aminoalkyl methacrylate copolymer E having enhanced solubility prepared by lyophilization

The procedure as described in Example 3 was repeated, except that the amount of hydrochloric acid was 8.2 mL, to prepare aminoalkyl methacrylate copolymer E having a solubility enhanced with acid.

Example 8: Aminoalkyl methacrylate copolymer E having enhanced solubility prepared by lyophilization

Aminoalkyl methacrylate copolymer E (product name: Eudragit E, Degussa) (5 g), citric acid monohydrate (Kanto Chemical Co., Inc.) (1 g), and purified water (125 mL) were mixed, and stirred until the aminoalkyl methacrylate copolymer E was dissolved. The resulting solution was lyophilized using a freeze dryer FD-81 (Tokyo Rikakiki Co,. Ltd.) to prepare aminoalkyl methacrylate copolymer E having a solubility enhanced with acid.

Example 9: Solid dispersion consisting of compound A and aminoalkyl methacrylate copolymer E having enhanced solubility prepared by spray-drying

Compound A (1.5 g), the aminoalkyl methacrylate copolymer E having a solubility enhanced with acid prepared in Example 2 (0.75 g), and sucrose (product name: frost sugar, Nissin Sugar Manufacturing Co., Ltd. ) (0.75 g) were pulverized using a planetary ball mill (LA-PO, Itoh Seisakujo Co., Ltd.) at a rotation rate of 200 rpm for 12 hours to prepare a solid dispersion in which the aminoalkyl methacrylate copolymer E was used as a carrier.

Comparative Example 1: Solid dispersion consisting of compound A and hydroxypropylmethylcellulose 2910

Compound A (1.5 g), hydroxypropylmethylcellulose 2910 (product name: TC-5E, Shin-Etsu Chemical Co., Ltd.) (0.75 g), and sucrose (product name: frost sugar, Nissin Sugar Manufacturing Co., Ltd.)(0.75 g) were pulverized using a planetary ball mill (LA-PO, Itoh Seisakujo Co., Ltd.) at a rotation rate of 200 rpm for 12 hours to prepare a solid dispersion in which hydroxypropylmethylcellulose 2910 was used as a carrier.

Example 10: Solid dispersion consisting of tacrolimus and aminoalkyl methacrylate copolymer E having enhanced solubility prepared by_lyophilization

Tacrolimus was dissolved in ethanol to prepare a 50 mg/mL tacrolimus solution. To 1 mL of this solution, 1 mL, of a 50 mg/mL aminoalkyl methacrylate copolymer E solution, which had been prepared by dissolving the aminoalkyl methacrylate copolymer E having a solubility enhanced with acid prepared in Example 4 in methanol, was added. This mixture was transferred to an agate mortar, and well kneaded with an agate pestle at room temperature until the solvent was completely evaporated, to prepare a solid dispersion in which the aminoalkyl methacrylate copolymer E having a solubility enhanced with acid was used as a carrier.

Example 11a Solid dispersion consisting of tacrolimus and aminoalkyl methacrylate copolymer E having enhanced solubility prepared by lyophilization

The procedure as described in Example 10 was repeated, except that the amount of aminoalkyl methacrylate copolymer E solution was 3 mL, to prepare a solid dispersion in which the aminoalkyl methacrylate copolymer E having a solubility enhanced with acid was used as a carrier.

Example 12: Solid dispersion consisting of tacrolimus and aminoalkyl methacrylate copolymer E having enhanced solubility prepared by lyophilization

The procedure as described in Example 10 was repeated, except that the amount of aminoalkyl methacrylate copolymer E solution was 5 mL, to prepare a solid dispersion in which the aminoalkyl methacrylate copolymer E having a solubility enhanced with acid was used as a carrier.

Comparative Example 2: Solid dispersion consisting of tacrolimus and hydroxypropylmethylcellulose 2910

Tacrolimus was dissolved in ethanol to prepare a 50 mg/mL tacrolimus solution. To 1 mL of this solution, 1 mL of a 50 mg/mL hydroxypropylmethylcellulose 2910 solution, which had been prepared by dissolving hydroxypropylmethylcellulose 2910 in a mixed solvent (methanol: dichloromethane = 1:1), was added. This mixture was transferred to an agate mortar, and well kneaded with an agate pestle at room temperature until the solvent was completely evaporated, to prepare a solid dispersion in which hydroxypropylmethylcellulose 2910 was used as a carrier.

Comparative Examples 3: Solid dispersion consisting of tacrolimus and hydroxypropylmethylcellulose 2910

The procedure as described in Comparative Example 2 was repeated, except that 3 mL of the hydroxypropylmethylcellulose 2910 solution was used, to prepare a solid dispersion in which hydroxypropylmethylcellulose 2910 was used as a carrier.

Example 13: Pharmaceutical preparation formulated by adding aminoalkyl methacrylate copolymer E having enhanced solubility prepared by lyophilization to solid dispersion consisting of tacrolimus and hydroxypropylmethylcellulose 2910

The solid dispersion consisting of tacrolimus and hydroxypropylmethylcellulose 2910 prepared in Comparative Example 3 (2 g) and the aminoalkyl methacrylate copolymer E having a solubility enhanced with acid prepared in Example 4 (0.5 g) were uniformly mixed in a mortar with a pestle, to prepare a formulation obtained by adding the aminoalkyl methacrylate copolymer E having an enhanced solubility to the solid dispersion consisting of tacrolimus and hydroxypropylmethylcellulose 2910.

Example 14: Pharmaceutical preparation formulated by adding aminoalkyl methacrylate copolymer E having enhanced solubility prepared by lyophilization to solid dispersion consisting of tacrolimus and hydroxypropylmethylcellulose 2910

The procedure as described in Example 13 was repeated, except that the amount of the aminoalkyl methacrylate copolymer E having a solubility enhanced with acid was 1.5 g, to prepare a formulation obtained by adding the aminoalkyl methacrylate copolymer E having an enhanced solubility to the solid dispersion consisting of tacrolimus and hydroxypropylmethylcellulose 2910.

Example 15: Pharmaceutical preparation consisting of_ tacrolimus and aminoalkyl methacrylate copolymer E having enhanced solubility prepared by lyophilization

The solid dispersion prepared in Example 12 consisting of tacrolimus and the aminoalkyl methacrylate copolymer E having a solubility enhanced with acid (100 mg) and lactose (Pharmatose, manufactured by DMV)(900 mg) were uniformly mixed in a mortar with a pestle. A pharmaceutical formulation was obtained by filling No. 2 HPMC capsules with a portion (30 mg) of the resulting mixture.

Experimental Example 1: Dissolution test using compound A

Each (50 mg) of the aminoalkyl methacrylate copolymer E having a solubility enhanced with acid prepared in Example 1, hydroxypropylmethylcellulose 2910 (TC-5E, Shin-Etsu Chemical Co., Ltd.), and copolyvidone (Kollidon VA-64, BASF) was dissolved in 500 mL of a second fluid for disintegrating test used in a disintegrating test described in the Japanese Pharmacopoeia fifteenth edition. For comparison, 500 mL of a second fluid for disintegrating test used in a disintegrating test described in the Japanese Pharmacopoeia fifteenth edition without such additives was prepared. To each vessel, 2 mL of a solution of compound A (25 mg/mL) in acetonitrile was added, and an automated dissolution tester (manufactured by Toyama Sangyo Co., Ltd.) was used to carry out a reprecipitation test for compound A, in accordance with a dissolution test, method 2, described in the Japanese Pharmacopoeia. The ultraviolet absorbance of compound A was measured at a wavelength of 273 nm. The paddle rotation speed was 50 rpm. The percentage dissolved was calculated on the basis that an absorbance of 0.576 when measured using a 1 mm cell was regarded as a concentration of 100 $\mu$g/mL (compound A).

Results and discussions

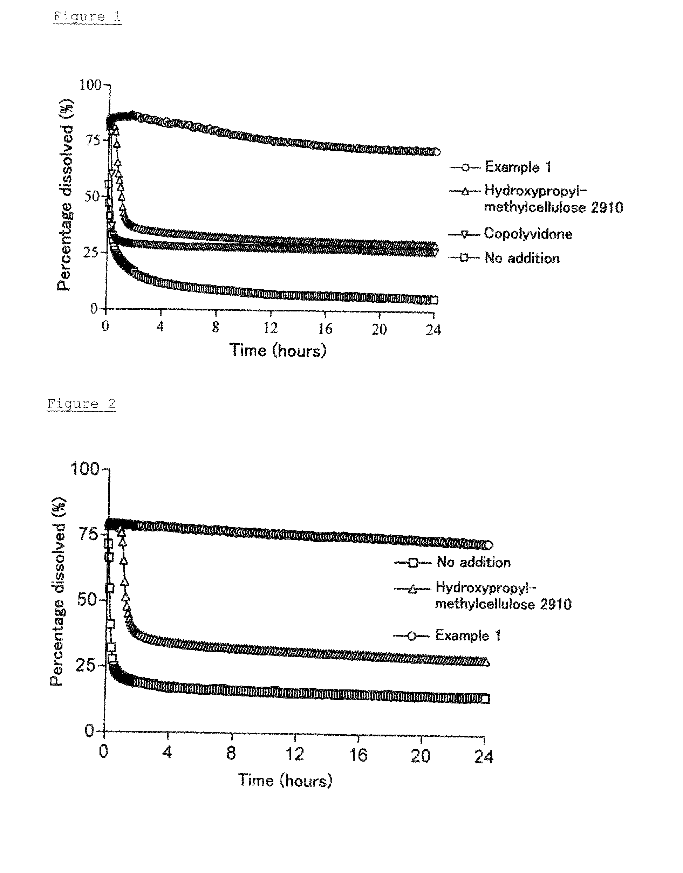

The result of Experimental Example 1 is shown in Figure 1. Compound A dissolved using acetonitrile was reprecipitated immediately after the beginning of the test, in the absence of additives. When hydroxypropylmethylcellulose 2910 or copolyvidone was added, the reprecipitation of compound A was inhibited in comparison with in the absence of additives. However, the inhibition effect thereof was small, and the percentage dissolved after 2 hours from the beginning of the test was approximately 30%. By contrast, when the aminoalkyl methacrylate copolymer E having a solubility enhanced with acid prepared in Example 1 was added, a percentage dissolved of approximately 75% was maintained even after 24 hours from the beginning of the test, and therefore, it was found that the solubility and the solubilized state of compound A were maintained for a long time by the aminoalkyl methacrylate copolymer E having a solubility enhanced with acid.

Experimental Example 2: Dissolution test using compound A

Each (50 mg) of the aminoalkyl methacrylate copolymer E having a solubility enhanced with acid prepared in Example 1, and hydroxypropylmethylcellulose 2910 (TC-5E, Shin-Etsu Chemical Co., Ltd.) was dissolved in 500 mL of a second fluid for dissolution test used in a dissolution test described in the Japanese Pharmacopoeia fifteenth edition. For comparison, 500 mL of a second fluid for dissolution test used in a dissolution test described in the Japanese Pharmacopoeia fifteenth edition without such additives was prepared. To each vessel, 2 mL of a solution of compound A (25 mg/mL) in polyethylene glycol 400 (Kanto Chemical Co., Inc.) was added, and an automated dissolution tester (manufactured by Toyama Sangyo Co., Ltd.) was used to carry out a reprecipitation test for compound A, in accordance with a dissolution test, method 2, described in the Japanese Pharmacopoeia. The ultraviolet absorbance of compound A was measured at a wavelength of 273 nm. The paddle rotation speed was 50 rpm. The percentage dissolved was calculated on the basis that an absorbance of 0.576 when measured using a 1 mm cell was regarded as a concentration of 100 $\mu$g/mL (compound A).

Results and discussions

The result of Experimental Example 2 is shown in Figure 2. Compound A dissolved using polyethylene glycol 400 as a surfactant was reprecipitated immediately after the beginning of the test, in the absence of additives. When hydroxypropylmethylcellulose 2910 was added, the reprecipitation of compound A was inhibited in comparison with in the absence of additives. However, the inhibition effect thereof was small, and the percentage dissolved after 2 hours from the beginning of the test was approximately 30%. By contrast, when the aminoalkyl methacrylate copolymer E having a solubility enhanced with acid prepared in Example 1 was added, a percentage dissolved of approximately 75% was maintained even after 24 hours from the beginning of the test, and therefore, it was found that the solubility and the solubilized state of compound A were maintained for a long time by the aminoalkyl methacrylate copolymer E having a solubility enhanced with acid.

Experimental Example 3: Dissolution test using compound A

Each of the aminoalkyl methacrylate copolymers E having a solubility enhanced with acid prepared in Examples 5, 6, and 7 was dissolved in 500 mL of a second fluid for dissolution test used in a dissolution test described in the Japanese Pharmacopoeia fifteenth edition.
To each vessel, 2 mL of a solution of compound A (25 mg/mL) in acetonitrile was added, and an automated dissolution tester (Toyama Sangyo Co., Ltd.) was used to carry out a reprecipitation test for compound A, in accordance with a dissolution test, method 2, described in the Japanese Pharmacopoeia. The absorbance of compound A was measured at a wavelength of 273 nm. The paddle rotation speed was 50 rpm. The percentage dissolved was calculated on the basis that an absorbance of 0.576 when measured using a 1 mm cell was regarded as a concentration of 100 $\mu$q/ml, (compound A).

Results and discussions

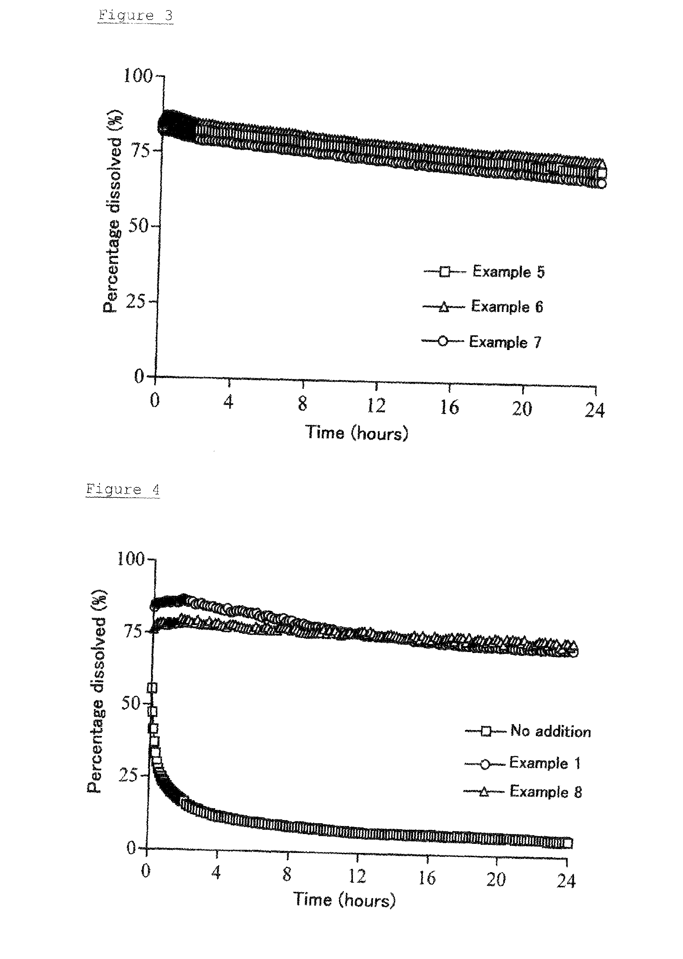

The result of Experimental Example 3 is shown in Figure 3. In Examples 5, 6, and 7, amounts of neutralization in each aminoalkyl methacrylate copolymers E were approximately 76, 93, and 127%, respectively. In every Example, a high solubility was maintained for a long time, and therefore, it was found that the solubility and the solubilized state of compound A were maintained for a long time by the aminoalkyl methacrylate copolymer E having a solubility enhanced with acid, with respect to every amount of neutralization.

Experimental Example 4: Dissolution test using compound A

Each (50 mg) of the aminoalkyl methacrylate copolymers E having a solubility enhanced with acid prepared in Examples 1 and 8 was dissolved in 500 mL of a second fluid for disintegrating test used in a disintegrating test described in the Japanese Pharmacopoeia fifteenth edition. For comparison, 500 mL of a second fluid for disintegrating test used in a disintegrating test described in the Japanese Pharmacopoeia fifteenth edition without such additives was prepared.
To each vessel, 2 mL of a solution of compound A (25 mg/mL) in acetonitrile was added, and an automated dissolution tester (Toyama Sangyo Co., Ltd.) was used to carry out a reprecipitation test for compound A, in accordance with a dissolution test, method 2, described in the Japanese Pharmacopoeia. The ultraviolet absorbance of compound A was measured at a wavelength of 273 nm. The paddle rotation speed was 50 rpm. The percentage dissolved was calculated on the basis that an absorbance of 0.576 when measured using a 1 mm cell was regarded as a concentration of 100 $\mu$g/mL (compound A) .

Results and discussions

The result of Experimental Example 4 is shown in Figure 4, which includes the result of Experimental Example 1 (no addition and Example 1) for comparison. In Example 8, the solubility of aminoalkyl methacrylate copolymer E was enhanced by using citric acid as acid. The solubility of compound A was maintained for a long time, in a similar profile as observed in Example 1 in which the solubility was enhanced with hydrochloric acid. It was found that the solubility and the solubilized state of compound A were maintained for a long time by the aminoalkyl methacrylate copolymer E having a solubility enhanced with acid, independently of the kind of acid.

Experimental Example 5: Dissolution test using compound B

Each (50 mg) of the aminoalkyl methacrylate copolymer E having a solubility enhanced with acid prepared in Example 3, and hydroxypropylmethylcellulose 2910 (TC-5E, Shin-Etsu Chemical Co., Ltd.) was dissolved in 500 mL of a second fluid for disintegrating test used in a disintegrating test described in the Japanese Pharmacopoeia fifteenth edition. For comparison, 500 mL of a second fluid for disintegrating test used in a disintegrating test described in the Japanese Pharmacopoeia fifteenth edition without such additives was prepared.
To each vessel, 4 mL of a solution of compound B (2.5 mg/mL) in acetone was added, and an automated dissolution tester (Toyama Sangyo Co., Ltd.) was used to carry out a reprecipitation test for compound B, in accordance with a dissolution test, method 2, described in the Japanese Pharmacopoeia. The absorbance of compound B was measured at a wavelength of 314 nm. The paddle rotation speed was 50 rpm. The percentage dissolved was calculated on the basis that an absorbance of 0.535 when measured using a 5 mm cell was regarded as a concentration of 20 $\mu$g/mL (compound B).

Results and discussions

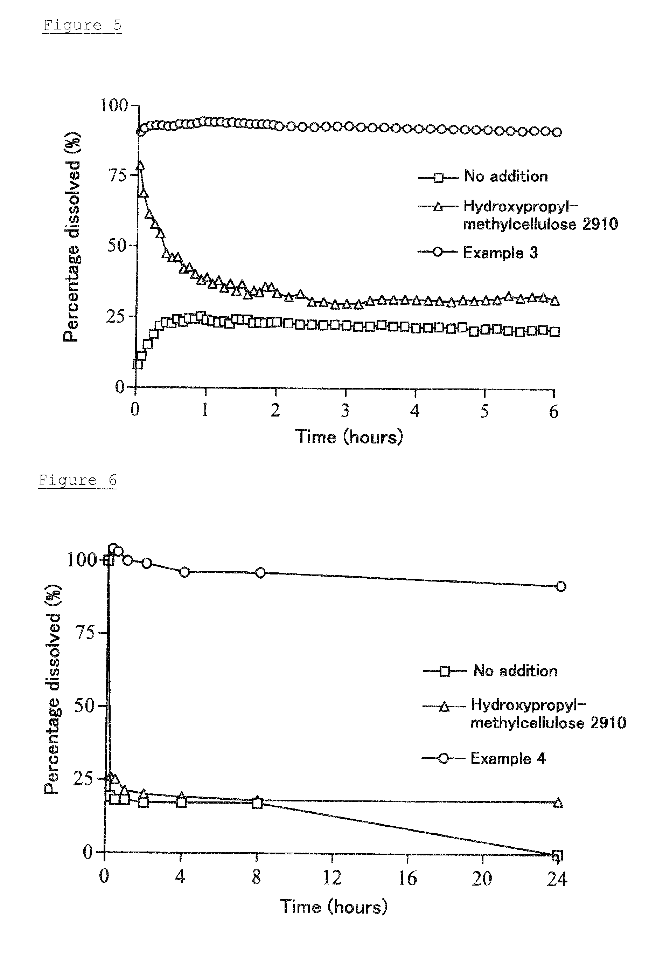

The result of Experimental Example 5 is shown in Figure 5. Compound B dissolved using acetone was reprecipitated immediately after the beginning of the test, in the absence of additives. When hydroxypropylmethylcellulose 2910 was added, the reprecipitation of compound B was inhibited in comparison with that in the absence of additives. However, the inhibition effect thereof was small, and the percentage dissolved after 2 hours from the beginning of the test was approximately 30%. By contrast, when the aminoalkyl methacrylate copolymer E having a solubility enhanced with acid prepared in Example 3 was added, a percentage dissolved of approximately 90% was maintained even after 6 hours from the beginning of the test, and therefore, it was found that the solubility and the solubilized state of compound B were maintained for a long time by the aminoalkyl methacrylate copolymer E having a solubility enhanced with acid.

Experimental Example 6: Dissolution test using tacrolimus

Each (300 mg) of the aminoalkyl methacrylate copolymer E having a solubility enhanced with acid prepared in Example 4, and hydroxypropylmethylcellulose 2910 (TC-5E, Shin-Etsu Chemical Co., Ltd.) was dissolved in 300 mL of a second fluid for disintegrating test used in a disintegrating test described in the Japanese Pharmacopoeia fifteenth edition. For comparison, 300 mL of a second fluid for disintegrating test used in a disintegrating test described in the Japanese Pharmacopoeia fifteenth edition without such additives was prepared.
To each vessel, 1 mL of a solution of tacrolimus (100 mg/mL) in ethanol was added. Samples were taken at regular intervals, and centrifuged at 3000 rpm for 15 minutes. Tacrolimus dissolved in each supernatant was measured by a high performance liquid chromatography.

Results and discussions

The result of Experimental Example 6 is shown in Figure 6. Tacrolimus dissolved using ethanol was reprecipitated immediately after the beginning of the test, in the absence of additives. When hydroxypropylmethylcellulose 2910 was added, the reprecipitation of tacrolimus was inhibited in comparison with that in the absence of additives. However, the inhibition effect thereof was small, and the percentage dissolved after 2 hours from the beginning of the test was approximately 20%. By contrast, when the aminoalkyl methacrylate copolymer E having a solubility enhanced with acid prepared in Example 4 was added, a percentage dissolved of 90% or more was maintained even after 24 hours from the beginning of the test, and therefore, it was found that the solubility and the solubilized state of tacrolimus were maintained for a long time by the aminoalkyl methacrylate copolymer E having a solubility enhanced with acid.

Experimental Example 7: Dissolution test using solid dispersion of compound A

Each (400 mg; equivalent to 200 mg of compound A) of the solid dispersions of compound A prepared in Example 9 and Comparative Example 1, or compound A (200 mg) was added to 900 mL of a second fluid for disintegrating test used in a disintegrating test described in the Japanese Pharmacopoeia fifteenth edition, in each vessel. An automated dissolution tester (Toyama Sangyo Co., Ltd.) was used to carry out a reprecipitation test for compound A, in accordance with a dissolution test, method 2, described in the Japanese Pharmacopoeia. The absorbance of compound A was measured at a wavelength of 273 nm. The paddle rotation speed was 50 rpm. The percentage dissolved was calculated on the basis that an absorbance of 1.28 when measured using a 1 mm cell was regarded as a concentration of 222 $\mu$g/mL (compound A).

Results and discussions

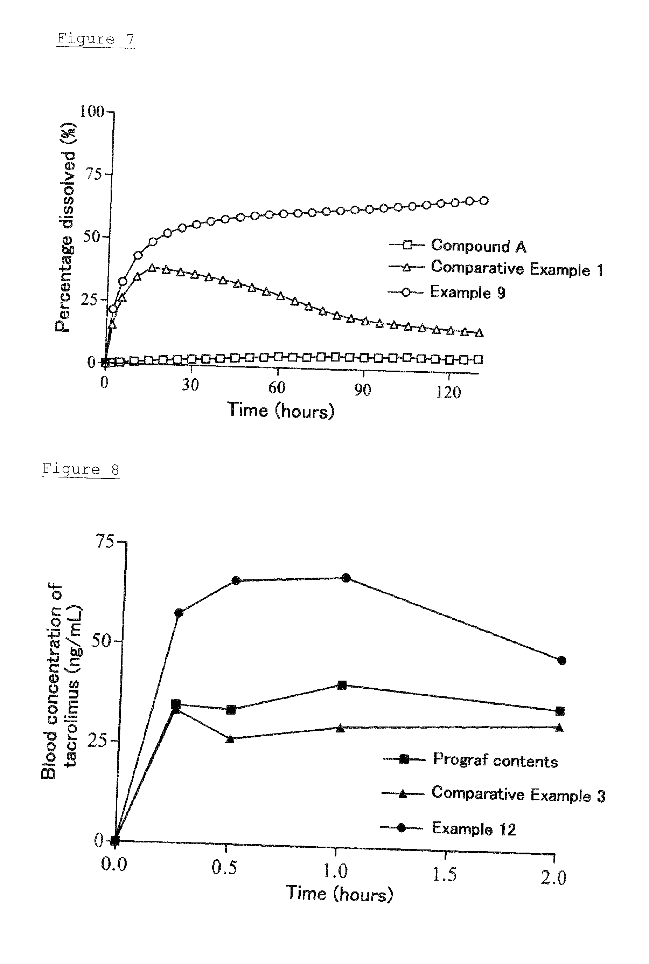

The result of Experimental Example 7 is shown in Figure 7. Both the solid dispersions of compound A prepared by dry pulverization in Comparative Example 1 and Example 9 exhibited a high solubility, in comparison with compound A alone. However, with respect to the solid dispersion prepared in Comparative Example 1, the solubility was temporarily enhanced, but compound A was gradually reprecipitated, and the solubility was lowered to approximately 20% after 2 hours from the beginning of the test. By contrast, the solid dispersion prepared in Example 9 exhibited a high solubility even after 2 hours from the beginning of the test, and therefore, it was found that the aminoalkyl methacrylate copolymer E having a solubility enhanced with acid accomplished a highly supersaturated state of compound A, and maintained the solubility of compound A for a long time.

Experimental Example 8: Measurement of solubility and maintenance time of tacrolimus solid dispersion

Each of the solid dispersions of tacrolimus prepared in Examples 10, 11, and 12, and Comparative Examples 2 and 3 was added to a second fluid for disintegrating test used in a disintegrating test described in the Japanese Pharmacopoeia fifteenth edition, and stirred using a vortex. Samples were taken at predetermined times, and centrifuged at 3000 rpm for 15 minutes. Each supernatant was measured by a high performance liquid chromatography.
[Table 1]

| Time | Solubility of tacrolimus ($\mu$g/mL) | | | | |
|---|---|---|---|---|---|
| | Comp.Ex.2 | Comp.Ex.3 | Ex.10 | Ex.11 | Ex.12 |
| 0.25 hour | 79 | 84 | 91 | 1205 | 4787 |
| 6 hours | 79 | 84 | 100 | 1170 | 4437 |
| 24 hours | - | 84 | - | 1099 | 3937 |

Results and discussions

The result of Experimental Example 8 is shown in Table 1. The solubility of each of the solid dispersions prepared in Comparative Examples 2 and 3 was approximately 80 $\mu$g/mL, which was similar to each other regardless of the contents of hydroxypropylmethylcellulose 2910. By contrast, with respect to Examples 10, 11, and 12, an increased content of the aminoalkyl methacrylate copolymer E having a solubility enhanced with acid elevated the solubility. In the solid dispersion prepared in Example 12, the solubility after 0.25 hour was 4787 $\mu$g/mL, which was 60 times or more higher than those of Comparative Examples, and a high solubility of 3937 $\mu$g/mL was observed even after 24 hours. It was found that the Aminoalkyl methacrylate copolymer E having a solubility enhanced with acid accomplished a highly supersaturated state of tacrolimus, and maintained the solubility of tacrolimus for a long time.

Experimental Example 9: Measurement of solubility of preparation formulated by adding aminoalkyl methacrylate copolymer E having enhanced solubility prepared by lyophilization to solid dispersion consisting of tacrolimus and hydroxypropylmethylcellulose 2910

Each of the solid dispersions of tacrolimus prepared in Examples 13 and 14, and Comparative Example 3 was added to a second fluid for disintegrating test used in a disintegrating test described in the Japanese Pharmacopoeia fifteenth edition, and stirred using a vortex. Samples were taken at predetermined times, and centrifuged at 3000 rpm for 15 minutes. Each supernatant was measured by a high performance liquid chromatography.
[Table 2]

| Time | Solubility ($\mu$g/mL) | | |
|---|---|---|---|
| | Comp.Ex.3 | Ex.13 | Ex.14 |
| 0.25 hour | 83.8 | 503.9 | 912.1 |
| 4 hours | - | 652.4 | 1183.4 |
| 6 hours | 87.9 | 661.5 | 1193.0 |

Results and discussions

The result of Experimental Example 9 is shown in Table 2. Whereas the solubility of tacrolimus was approximately 80 to 90 $\mu$g/mL in Comparative Example 3, the solubilities of tacrolimus after 0.25 hour were 503.9 $\mu$g/mL and 912.1 $\mu$g/mL in Examples 13 and 14, respectively, and the solubilities were enormously enhanced. The solubilities of tacrolimus after 6 hours were 661.5 $\mu$g/mL and 1193.0 $\mu$g/mh in Examples 13 and 14, respectively, and the solubilities were gradually further enhanced. It was found that the solubility of tacrolimus was enhanced by the addition of aminoalkyl methacrylate copolymer E.

Experimental Example 10: Administration of tacrolimus solid dispersion to rats (Effects in vivo)

Each (equivalent to 1.5 mg of tacrolimus per body) of the solid dispersions of tacrolimus prepared in Example 12 and Comparative Example 3, and the contents of Prograf capsules (product name) were suspended to a second fluid for disintegrating test used in a disintegrating test described in the Japanese Pharmacopoeia fifteenth edition, to prepare 1 mL of each test solution. After male Wistar rats (7-week-old, weight: 200 to 250 g, Charles River Laboratories Japan, Inc.) were fasted for 16 hours or more, each test solution was forcefully orally administered using a catheter to rats. The rats were dissected under anesthesia with ether, and blood samples (approximately 0.7 mL) were collected from a jugular vein, using heparinized syringes (26G needle), after 0.25, 0.5, 1, and 2 hours from the administration (n=2). Blood concentrations of tacrolimus in the blood samples were measured with LC-MS/MS (TSQ7000, ThermoQuest). From the time course of obtained blood concentrations, a maximum blood concentration (Cmax) and an area under the blood concentration versus time curve (AUC) were calculated.
[Table 3]

|  | Prograf contents | Comp.Ex.3 | Ex.12 |
|---|---|---|---|
| Cmax(ng/mL) | 40.8 | 33.5 | 67.7 |
| AUC 0-2h(ng/h/mL) | 70.0 | 56.9 | 114.3 |
| (Average, n=2) | | | |

Results and discussions

The result of Experimental Example 10 is shown in Table 3 and Figure 8. The solid dispersion prepared in Example 12 exhibited higher Cmax and AUC values, in comparison with the contents of Prograf capsules (product name) and the solid dispersion prepared in Comparative Example 3. It was found that not only the solubility of tacrolimus, but also the absorption thereof in vivo was enhanced in the solid dispersion of tacrolimus containing the aminoalkyl methacrylate copolymer E having a solubility enhanced with acid.

Experimental Example 11: Administration of tacrolimus solid dispersion to dogs (Effects in vivo)

Male beagle dogs (approximately 20-month-old, weight: 8 to 15 kg, Nosan Corporation) were fasted for 16 hours, and primperan (0.25 mL/body) was intramuscularly administered to the dogs for preventing vomition, approximately 1 hour before an administration of test drugs. The capsule prepared in Example 15 was forcefully orally administered, and 30 mL of water was immediately orally administered. As a control, Prograf capsules (product name, 0.5 mg) were used. The dogs were not allowed to take water from approximately 30 minutes before the oral administration, and were allowed to freely take water after a blood collection at 2 hours later from the oral administration. The blood samples (approximately 2 mL) were collected before the oral administration, and after 0.25, 0.5, 0.75, 1, 2, 3, 4, and 6 hours from the oral administration (n=3). Blood concentrations of tacrolimus in the blood samples were measured with LC-MS/MS (TSQ7000, ThermoQuest). From the time course of obtained blood concentrations, a maximum blood concentration (Cmax) and an area under the blood concentration versus time curve (AUC) were calculated.
[Table 4]

|  | Prograf | Ex.15 |
|---|---|---|
| Cmax(ng/mL) | 4.8 | 11.2 |
| AUC 0-6h(ng/h/mL) | 7.3 | 23.7 |
| (Average, n=3) | | |

Results and discussions

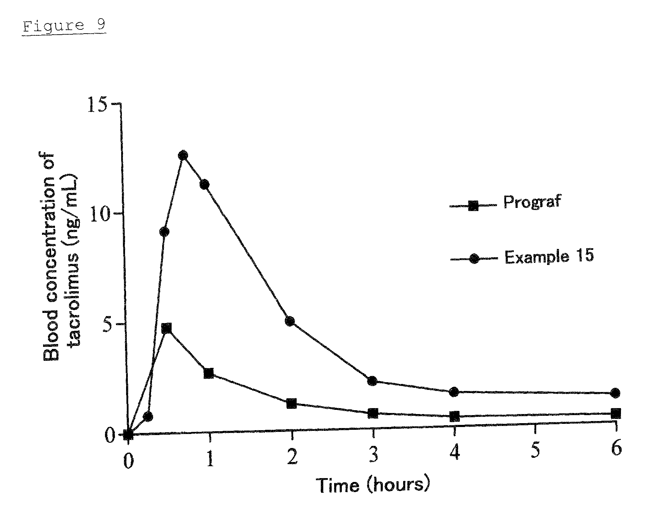

The result of Experimental Example 11 is shown in Table 4 and Figure 9. With respect to the solid dispersion prepared in Example 16, Cmax and AUC values were approximately 2 times and 3 times in comparison with those of Prograf, respectively, and the solid dispersion proved effective for enhanced absorption. It was found that not only the solubility of tacrolimus, but also the absorption thereof in vivo was enhanced in the solid dispersion of tacrolimus containing the aminoalkyl methacrylate copolymer E having a solubility enhanced with acid.

INDUSTRIAL APPLICABILITY

According to the present invention, an unevenness of absorption when a poorly-soluble drug is administered can be reduced, and sufficient absorption can be maintained, by maintaining the solubility and/or the solubilized state of a poorly-soluble drug for a long time. Therefore, a compound which exhibits a pharmacological activity against various diseases, but is not absorbed due to a low solubility to water can be developed as a drug, by applying the present invention to such a compound. As a result, new drugs can be placed on the market, and the present invention is expected to contribute to the development of industry.

As above, the present invention was explained with reference to particular embodiments, but modifications and improvements obvious to those skilled in the art are included in the scope of the present invention.

**Claims**

1.   A pharmaceutical composition comprising aminoalkyl methacrylate copolymer E, an acidic substance, and a poorly-soluble drug, wherein the aminoalkyl methacrylate copolymer E and the acidic substance are uniformly mixed, and solubility of the poorly-soluble drug is maintained for at least 30 minutes.

2.   The pharmaceutical composition according to claim 1, wherein the poorly-soluble drug is an amorphous state.

3.   The pharmaceutical composition according to claim 1, wherein the poorly-soluble drug is a solid dispersion.

4.   The pharmaceutical composition according to claim 1, wherein the poorly-soluble drug is dissolved in a solvent selected from an organic solvent, a surfactant, or an oily substance, or a mixture of one, or two or more of the solvents.

5.   The pharmaceutical composition according to claim 1, wherein the poorly-soluble drug is tacrolimus.

6.   Use of aminoalkyl methacrylate copolymer E which is uniformly mixed with an acidic substance, in the manufacture of a pharmaceutical composition for inhibiting reprecipitation of a poorly-soluble drug.

7.   Use of aminoalkyl methacrylate copolymer E which is uniformly mixed with an acidic substance according to claim 6, wherein the poorly-soluble drug is solubilized.

8.   A method of solubilizing a poorly-soluble drug, by using aminoalkyl methacrylate copolymer E which is uniformly mixed with an acidic substance.

9.   A method of inhibiting reprecipitation of a poorly-soluble drug, by using aminoalkyl methacrylate copolymer E which is uniformly mixed with an acidic substance.

10.  A method of maintaining a maximum percentage dissolved of a poorly-soluble drug within 90% to 100% for at least 30 minutes, when (1) a dissolution test is carried out by adding, to a dissolution test fluid, aminoalkyl methacrylate copolymer E which is uniformly mixed with an acidic substance, followed by a solubilized poorly-soluble drug or a dissolved poorly-soluble drug, or (2) a dissolution test is carried out by adding aminoalkyl methacrylate copolymer E which is uniformly mixed with an acidic substance, and a solid dispersion containing a poorly-soluble drug, to a dissolution test fluid.

11.  A process of manufacturing a pharmaceutical composition for maintaining solubility and/or a solubilized state of a poorly-soluble drug for at least 30 minutes, comprising the steps of:

preparing aminoalkyl methacrylate copolymer E which is uniformly mixed with an acidic substance by a method selected from:

(1) a method in which the acidic substance and the aminoalkyl methacrylate copolymer E are dissolved in water or a solvent, and the obtained liquid is spray-dried,
(2) a method in which the acidic substance and the aminoalkyl methacrylate copolymer E are dissolved in water or a solvent, and the obtained liquid is lyophilized, or

(3) a method in which the acidic substance and the aminoalkyl methacrylate copolymer E are dissolved in water or a solvent, and the obtained liquid is dried by evaporating the water or solvent therefrom; and mixing the aminoalkyl methacrylate copolymer E with a solubilized poorly-soluble drug or a dissolved poorly-soluble drug.

Figure 1

Figure 2

Figure 3

Figure 4

Figure 5

Figure 6

Figure 7

Figure 8

Figure 9

## INTERNATIONAL SEARCH REPORT

| International application No. |
|---|
| PCT/JP2007/074998 |

A. CLASSIFICATION OF SUBJECT MATTER
*A61K47/32*(2006.01)i

According to International Patent Classification (IPC) or to both national classification and IPC

B. FIELDS SEARCHED

Minimum documentation searched (classification system followed by classification symbols)
A61K47/32

Documentation searched other than minimum documentation to the extent that such documents are included in the fields searched

| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN), MEDLINE(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2002/005786 A1 (YAMANOUCHI PHARMACEUTICAL CO., LTD.),<br>24 January, 2002 (24.01.02),<br>All references; particularly, pages 10,<br>12 to 18, 31; examples 2, 5<br>& AU 200171055 A     & US 2002/150624 A1<br>& EP 1302201 A1 | 1,6-11<br>2-5 |
| Y | JP 2000-095708 A (HOFFMANN LA ROCHE & CO AG F),<br>04 April, 2000 (04.04.00),<br>All references; particularly, Par. Nos. [0011],<br>[0018], [0026]<br>& EP 988863 A2     & AU 9948807 A<br>& NO 9904583 A | 2-4 |

|☒| Further documents are listed in the continuation of Box C. | |☐| See patent family annex. |

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>01 April, 2008 (01.04.08) | Date of mailing of the international search report<br>15 April, 2008 (15.04.08) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2007/074998

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 03-074396 A  (MERCIAN CORP.),<br>28 March, 1991 (28.03.91),<br>All references; particularly, Claims<br>& EP 413299 A              & CA 2022772 A<br>& CN 1049451 A | 2-4 |
| Y | "Prograf Chushaeki 5mg", Tenpu Bunsho, 2006.10,<br>page 6 | 5 |
| A | WO 2003/059389 A1  (YAMANOUCHI PHARMACEUTICAL<br>CO., LTD.),<br>24 July, 2003 (24.07.03),<br>& US 2003/175351 A1     & AU 2003201886 A1<br>& EP 1466626 A1 | 1-11 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2000095708 A
- JP 2005500313 A
- WO 0205786 A
- WO 94006414 A
- WO 01078686 A
- WO 9520380 A

### Non-patent literature cited in the description

- **Germany et al.** Increasing the solubility of Felodipine by preparing solid dispersions with melt extrusion. *Kathrin Nollenberger,* 2006